# FASCICULE DE BREVET EUROPEEN

(11) **EP 1 355 577 B1**
(45) Date de publication et mention de la délivrance du brevet: **05.11.2008**
(21) Numéro de dépôt: 01272766.5
(22) Date de dépôt: 24.12.2001
(51) Int. Cl.: A61B 17/54

(54) **DISPOSITIF DE DERMABRASION**
HAUTABRASIONSVORRICHTUNG
DERMABRASION DEVICE

(30) Priorité: 28.12.2000 EP 00811254
(43) Date de publication de la demande: 29.10.2003
(73) Titulaire: Bernaz, Gabriel, 1227 Carouge (CH)
(72) Inventeur: Bernaz, Gabriel, 1227 Carouge (CH)
(74) Mandataire: Cronin, Brian Harold John
(86) Numéro de dépôt international: PCT/IB2001/002665
(87) Numéro de publication internationale: WO 2002/053046

(56) Documents cités:
- DE-A- 3 740 902
- US-A- 2 867 214
- US-A- 2 917 056
- US-A- 3 169 536
- US-A- 4 643 207
- US-A- 6 139 553

## Description

### DOMAINE TECHNIQUE

L'invention se rapporte à la dermabrasion de la peau par effleurage de la peau avec un abrasif, en particulier pour la microépidermabrasion de la peau, c'est-à-dire la microabrasion de la couche superficielle de la peau, ou épiderme, qui protège la partie profonde de la peau, ou derme, formée de tissu conjonctif contenant des vaisseaux, des nerfs et des follicules.

### ETAT DE LA TECHNIQUE

La microdermabrasion par la projection des microcristaux d'hydroxyde d'aluminium est connue par exemple par "La microdermabrasion en pratique" par François Mahuzier (http://www.mahuzier.com) ou encore par "La Dermabrasion" par Dr. André Camirand (http://www.drcamirand.com), ou encore par "Dermabrasion & Dermaplaning" par la Société canadienne de chirurgie plastique et esthétique (http://www.smartnet.ca). Ces articles mentionnent aussi la dermabrasion par des grattements avec une brosse métallique, ou par un burin contenant des particules de diamant, alors que le dermaplaning emploie un instrument appelé dermatome qui comporte une lame oscillante pour déloger des couches de surface de la peau.

La microdermabrasion par la projection des microcristaux de corundum est également décrite dans la publication EP-A-0 806 184.

La microdermabrasion par la projection d'un liquide est connue du brevet US 5,562,643, alors que le brevet US 5,562,643 décrit la microdermabrasion par l'emploi d'un outil à ultrasons, après l'application d'un anesthésique.

Le brevet US 5,562,643 décrit un appareil pour la microdermabrasion chirurgicale comportant une pluralité de feuilles flexibles à bord abrasif tournant autour d'un moyeu pour venir frapper la peau par la force centrifuge.

Ces appareils et procédés de dermabrasion sont destinés essentiellement à l'abrasion de la peau jusqu'au derme, et nécessitent en général une intervention chirurgicale.

Le traitement cosmétique de l'épiderme utilisant un tampon abrasif maniable du type Scotch-Brite^{™} est connu du brevet US 6,017,351, principalement dans le but d'enlever un excédant de peau morte.

Le brevet US 4,438,767 décrit une lame maniable d'exfoliation, également pour enlever un excédant de peau morte.

Le brevet US 4,643,207 concerne un dispositif de manucure destiné à abrader la cuticule par un abrasif oscillant, et qui comporte un organe de guidage qui pénètre sous le cuticule afin de limiter la pénétration de l'abrasif oscillant.

Le brevet US 2,917,056 décrit une plaque oscillante comme accessoire d'un rasoir électrique.

Le brevet US 6,139,553 décrit un dispositif pour le traitement du visage, comportant un disque rotatif abrasif, notamment pour faire pénétrer un produit traitant.

Le brevet US 2,867,214 décrit un appareil de dermaplanage à brosse rotative dont une exécution comporte un appui destiné à contrôler la profondeur de pénétration de la brosse rotative.

Le document DE 37 40 902 décrit un dispositif à cylindre rotatif pour le traitement des cornes de pied, dont le cylindre est entouré d'un support.

Aucun de ces appareils est destiné à la dermabrasion de la peau et en particulier la microépiderm-abrasion.

### EXPOSE DE L'INVENTION

La présente invention a pour objet un dispositif de dermabrasion de la peau par effleurage de la peau avec un abrasif, le dispositif comportant un boîtier maniable et des moyens d'entraînement en oscillation de l'abrasif. Ce dispositif est destiné en particulier aux traitements cosmétiques de l'épiderme appelés microépidermabrasion.

L'invention réside dans les caractéristiques de la partie caractérisante de la revendication 1.

On obtient ainsi une microabrasion de l'épiderme par l'oscillation de la surface abrasive arquée appliquée légèrement contre la peau grâce à la surface d'appui qui empêche toute blessure ou pénétration au-delà de l'épiderme. Le dispositif est aussi convivial à manier qu'un rasoir électrique, et permet la microépidermabrasion cosmétique de la peau pour différents traitements. Dès lors, il présente des avantages substantiels par rapport aux appareils connus de dermabrasion dont l'utilisation est limitée aux interventions chirurgicales ou analogues, et qui agissent jusque dans le derme. Par rapport aux appareils manuels pour traiter l'épiderme, le nouvel appareil permet un traitement automatisé et facile sur de grandes surfaces comme sur de petites surfaces, et permet de réaliser des traitements antirides et autres qui n'étaient pas faisables avec les appareils connus.

Pour promouvoir l'effet de microépidermabrasion, la surface abrasive arquée ne fait pas saillie par rapport à la surface d'appui, donc elle est soit au niveau de cette surface d'appui, soit avantageusement disposée en retrait par rapport à la surface d'appui, par exemple de 0 à 2 mm en retrait, usuellement au moins environ 1 à 1.5 mm, alors que le jeu latéral de la surface arquée par rapport aux bords de la surface d'appui est usuellement environ 1-4 mm de chaque côté.

Avantageusement, la surface abrasive est portée sur une pièce amovible en matière rigide ou souple sur le support oscillant. Ainsi, le dispositif peut comporter plusieurs pièces interchangeables chacune portant une surface abrasive différente et/ou ayant des dimensions différentes. Par exemple, on peut disposer de plusieurs pièces interchangeables portant une surface abrasive identique, mais de différentes longueurs adaptées au traitement de la peau de différentes parties du corps, et/ou plusieurs pièces interchangeables portant des surfaces abrasives différentes destinées à produire une microabrasion plus ou moins prononcée.

Le dispositif selon l'invention comporte avantageusement au moins une pièce amovible à double face montée de manière réversible sur le support oscillant, soit portant deux surfaces abrasives différentes, soit avec une surface abrasive arquée sur un côté et une surface lisse ou rugueuse de massage sur l'autre côté, notamment d'un profil convexe ou plate doté de stries ou autres protubérances. Cette dernière pièce amovible permet alors l'utilisation du dispositif de dermabrasion accessoirement pour effectuer un massage en inversant la pièce amovible pour utiliser sa surface rugueuse oscillante, notamment avec la surface d'appui enlevé et avec un mouvement d'oscillation et de vibration combiné, comme décrit plus loin.

La pièce porte-abrasif est soit en matière rigide, par exemple en matière plastique moulée, soit en matière souple par exemple une pièce en silicone souple permettant d'adapter la force d'application de l'abrasif sur la peau.

Dans une forme d'exécution, la surface d'appui est constituée par les bords d'un élément en U qui entourent la pièce portant la surface abrasive, cette pièce étant amovible par l'extrémité ouverte de l'élément en U de la surface d'appui, facilitant ainsi l'échange des pièces interchangeables. L'extrémité ouverte permet aussi l'inclinaison de l'appareil pour le traitement d'endroits enfoncés, comme des sillons de rides.

La surface d'appui du dispositif est normalement constituée soit par les bords d'un élément amovible sur le boîtier, soit par les bords du boîtier. Dans ce dernier cas, les bords de la surface d'appui constituant une partie intégrante du boîtier lequel est par exemple en matière thermoplastique moulée.

Un dispositif dont la surface d'appui est disposée sur un élément amovible présente la possibilité d'utiliser accessoirement le dispositif avec cet élément démonté pour le traitement des parties du corps ne nécessitant pas l'utilisation d'une surface d'appui. On peut citer notamment le manucure et le pédicure, également des traitements successifs de dermabrasion et de massage.

Avantageusement, les moyens d'entraînement de l'abrasif permettent la variation de la vitesse d'oscillation de la surface abrasive dont la vitesse d'oscillation peut être comprise entre 0.5 à 200 oscillations par seconde, usuellement entre 5-100 oscillations par seconde.

Dans une forme d'exécution, ces moyens d'entraînement comportent un étrier solidaire d'un levier monté pivotant dans le boîtier, l'étrier entourant un excentrique entraîné par l'arbre d'un moteur électrique, le support abrasif étant monté à l'extrémité pivotante du levier. Dans ce cas, le support abrasif oscille en balancier autour de son axe de pivotement, cet axe de pivotement étant de préférence disposé entre l'excentrique et l'extrémité pivotante du levier, de manière à ce que le rayon de pivotement soit réduit.

De manière générale, ce rayon de pivotement serait au maximum environ 20 mm, usuellement entre 10-15 mm. Pour assurer un bon effet de microépidermabrasion l'amplitude d'oscillation est usuellement au maximum environ 4 mm (2 mm de chaque côté).

Selon une forme d'exécution, l'axe d'oscillation de la surface arquée est incliné par rapport à l'axe du boîtier, à un angle jusqu'à 25°, par exemple environ 15° à 20°, ce qui facilite notamment sa maniabilité pour certains traitements.

Les formes d'exécution précitées peuvent comporter un moyen d'entraînement du support abrasif - ou toute autre support, notamment pour une surface rugueuse de massage - d'un mouvement d'oscillation combiné avec une vibration, ceci par un mouvement de va-et-vient perpendiculaire audit mouvement d'oscillation, simultanément avec ledit mouvement d'oscillation ou au lieu de celui-ci. Ceci permet l'utilisation accessoire du dispositif de dermabrasion pour effectuer un massage, notamment par l'emploi d'un accessoire de massage tel que la pièce amovible double-face précitée comportant sur une face une surface rugueuse de massage.

Dans une autre forme d'exécution, l'abrasif est disposé sur un support cylindrique oscillé par des moyens adéquats. Une telle configuration cylindrique permet de développer des surfaces oscillantes de grande taille, augmentant les possibilités d'application du dispositif. Avec un support abrasif cylindrique il est en outre possible de disposer de plusieurs zones abrasives écartées les unes par rapport aux autres autour de la périphérie du cylindre, chaque zone correspondant à un angle d'oscillation du cylindre. Le cylindre est alors indexable pour permettre la sélection de la zone abrasive accessible par la surface d'appui.

L'invention concerne également un procédé de traitement cosmétique de la peau par microépidermabrasion, utilisant le dispositif selon l'invention, de préférence après avoir appliqué auparavant à la surface de la peau à traiter un produit de nettoyage. Cette microépidermabrasion en effet expose le tissu de la peau et rend ce tissu de la peau plus apte à suivre divers traitements.

Un procédé de traitement cosmétique de la peau selon l'invention comporte avantageusement au préalable une microépidermabrasion au moyen de l'appareil inventif, suivi de l'application sur l'épiderme ainsi traité d'un produit traitant qu'on fait pénétrer dans le tissu de la peau par l'application d'un flux d'énergie électromagnétique haute fréquence et/ou par l'application de rayonnement électromagnétique laser, par exemple selon les brevets EP 0573618 et 0773744 et la demande de brevet WO/99/49800, ou par une source de lumière "normale".

Ce traitement est avantageusement un traitement antirides, autour des yeux, sur le front, autour de la bouche, etc. mais d'autres traitements sont aussi possible par exemple un traitement d'épilation au-dessus des lèvres ou autour des sourcils ou sur des surfaces plus larges. D'autres traitements sont le traitement des taches, des vergetures, de l'acné et des cicatrices. Il convient aussi pour des traitements sur le cuir-chevelu, notamment pour y faire pénétrer des produits de repousse de cheveux.

Enfin, l'invention concerne aussi l'utilisation du dispositif pour la microépidermabrasion de la peau aussi pour des traitements combinés microépidermabrasion et massage.

### BREVE DESCRIPTION DES DESSINS

D'autres caractéristiques de l'invention ressortiront à la lecture de la description qui suit, donnée à titre d'exemple, en se référant aux dessins dans lesquels :
la Figure 1 est une vue latérale en coupe axiale selon la ligne I-I de la Figure 2 d'une forme d'exécution du dispositif selon l'invention;
la Figure 2 est une vue schématique en plan et coupe de la Figure 1;
la Figure 3 est une vue schématique latérale en coupe, montrant l'appareil des Figures 1 et 2 en contact avec la peau;
la Figure 4 est une vue latérale en coupe axiale d'une exécution dont l'axe d'oscillation est incliné par rapport à l'axe du boîtier;
les Figures 5a et 5b sont des coupes de deux pièces support-abrasif à double face réversible;
la Figure 6 est un schéma comme la Figure 3 montrant la pièce réversible de la Figure 5b utilisée pour la dermabrasion; et
la Figure 7 est un schéma comme la Figure 6 montrant la pièce réversible de la Figure 5b utilisée pour le massage, avec la surface d'appui démonté.

### MODES DE REALISATION PREFERES

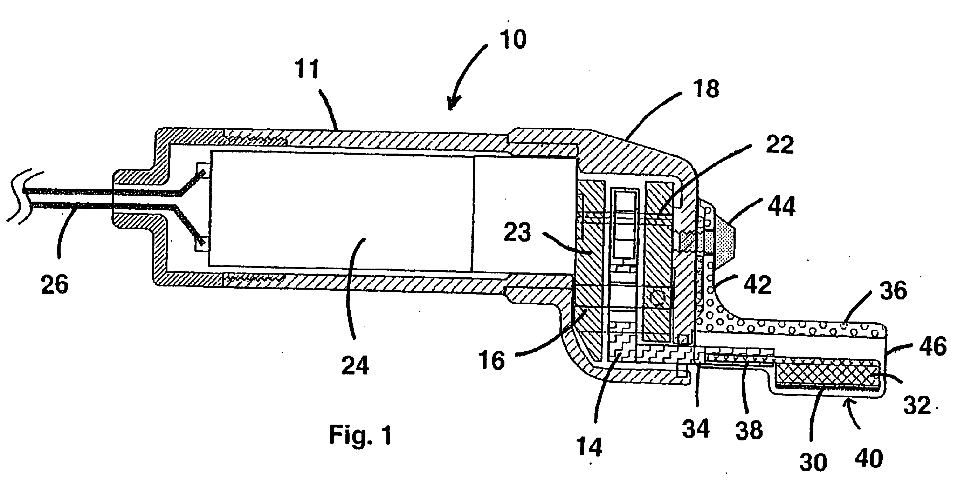

Les Figures 1 à 3 représentent un dispositif de dermabrasion de la peau comportant un boîtier maniable 10 et des moyens d'entraînement d'un abrasif 30. Dans cette forme d'exécution, les moyens d'entraînement comportent un étrier 12 solidaire d'un levier 14 monté pivotant autour d'un axe 16 dans un carter 18 monté à une extrémité du corps allongé du boîtier 10. Les deux bras de l'étrier 12 entourent un excentrique 20 entraîné par l'arbre 22 d'un moteur électrique 24 logé dans le corps allongé 11 du boîtier 10.

L'ensemble arbre 22, excentrique 20, levier 14, axe 16 est monté dans un corps monobloc 23 logé dans le carter 18.

La surface abrasive 30 est constituée d'une surface arquée d'une pièce allongée 32 retenue amoviblement sur un support 34 monté sur l'extrémité inférieure du levier 14, cette pièce 32 faisant saillie par l'extrémité avant du boîtier 10. L'axe de cette surface abrasive arquée 30 coïncide avec l'axe 16 de pivotement du levier 14. Ainsi, l'oscillation du levier 14 produit une oscillation en balancier de la surface abrasive arquée 30 autour de son axe dont le rayon de courbure se situe entre environ 1-3cm par exemple.

La pièce porte-abrasif 32 est par exemple en matière plastique moulée rigide, ou en silicone souple permettant d'adapter la force d'application de l'abrasif 30 sur la peau.

Le moteur 24 est alimenté par le secteur au moyen des fils 26 d'amenée de courant (et/ou par un accumulateur) et comporte un variateur de vitesse permettant de varier la vitesse de rotation de l'arbre 22 et excentrique 20, donc de la vitesse d'oscillation du levier 14 et de la surface abrasive 30. Cette surface abrasive 30 oscille de préférence à une vitesse entre 0.5 à 200 oscillations par seconde, variable progressivement ou par paliers, éventuellement à une seule vitesse d'oscillation dans cette gamme, usuellement entre 5 à 100 oscillations par seconde.

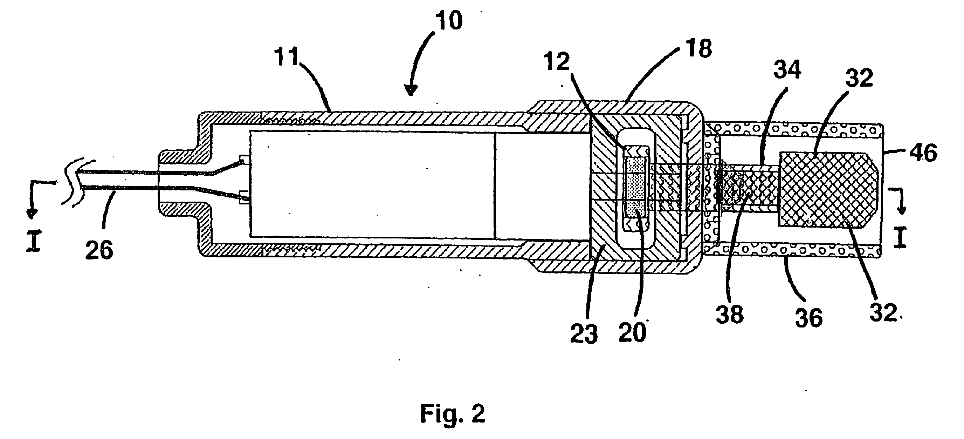

Le dispositif comporte en outre une surface d'appui 40 constituée par les bords d'un élément en U 36 qui entoure la pièce porte-abrasif 32. Cet élément 36 entoure trois côtés sur quatre de la surface abrasive oscillante 30 (voir la Figure 2). Cette pièce 32 comporte une tige 38 montée amoviblement par friction dans un logement correspondant du support oscillant 34.

L'élément en U 36 comporte une partie frontale 42 servant à solidariser cet élément 36 sur le carter 18 au moyen d'une vis 44, permettant aussi d'enlever cet élément 36 si désiré. Normalement cet élément 36 reste en place, car il est nécessaire pour la sécurité d'utilisation et pour assurer l'effleurage de l'abrasif oscillant sur la peau 50 suffisant pour effectuer une microépidermabrasion, sans risque de pénétration dans le derme 52.

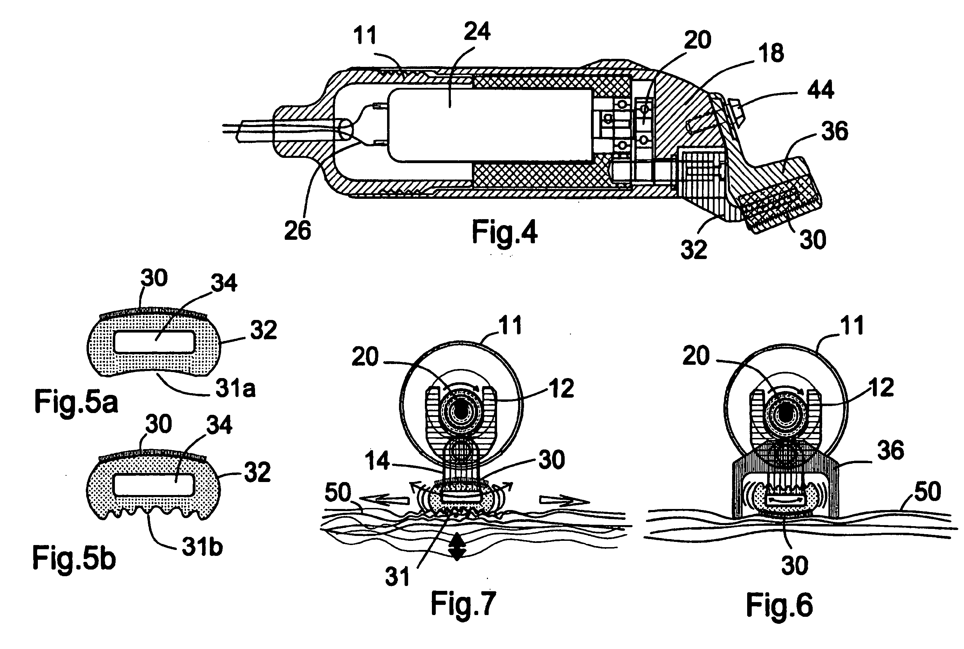

Lorsque l'élément 36 est enlevé, il est encore possible d'utiliser le dispositif non plus pour la microépidermabrasion mais pour le traitement abrasif des parties du corps ne nécessitant pas la protection conférée par la surface d'appui 40, par exemple pour le manucure et le pédicure, aussi pour le massage (Figure 7).

La pièce porte-abrasif 32 est montable et démontable par l'extrémité ouverte 46 de l'élément en U 36, facilitant ainsi l'échange des pièces 32 interchangeables. D'autres moyens de fixation amovible sont possible.

Le dispositif comporte avantageusement plusieurs pièces 32 interchangeables chacune portant une surface abrasive 30 différente, par exemple en poudre de saphir ou alumine, ou un autre abrasif ayant de manière générale une grosseur de grain usuellement dans la gamme 50-200, convenablement d'environ 80-120, et une dureté adaptées pour des traitements de microabrasion plus ou moins prononcée. De manière générale un abrasif d'un grain plus grossier ou plus fin sera utilisé avec une vitesse d'oscillation relativement basse ou haute, respectivement.

Les pièces porte-abrasifs 32 interchangeables peuvent aussi avoir des dimensions différentes, par exemple des longueurs différentes adaptées au traitement de la peau de différentes parties du corps.

Dans cet exemple, la surface abrasive arquée 30 est disposée en retrait par rapport à la surface d'appui 40, usuellement d'au moins environ 1 à 1.5 mm, alors que le jeu latéral de la surface arquée par rapport aux bords l'élément en U 36 est environ 1-2 mm de chaque côté.

L'utilisation de l'appareil est très commode car le boîtier 10 est maniable, comme un rasoir électrique. L'application de la surface d'appui 40 contre la peau 50 à traiter, autour de la région de la peau à traiter, permet l'effleurage de la peau 50 par la surface abrasive oscillante 30 entraîné par le moteur 24.

On obtient ainsi une microabrasion de l'épiderme 54 par l'oscillation de la surface abrasive arquée 30 appliquée légèrement contre la peau 50 grâce à la surface d'appui 40 qui empêche toute blessure ou pénétration au-delà de l'épiderme 54. De manière générale le dispositif est balayé sur la surface de la peau à traiter pendant un temps convenable, usuellement durant plusieurs minutes.

Normalement la surface arquée 30 sera appliquée à plat sur la peau 50, mais pour le traitement de sillons des rides et autres endroits enfoncés, il est possible d'incliner l'appareil (vers la droite de la Fig. 1) et travailler sur la pointe de l'abrasif, c'est-à-dire, l'extrémité à droite de la Fig. 1. L'angle de travail peut donc varier de 0° jusqu'à environ 45°.

La Figure 4, où les mêmes références désignent les mêmes éléments, montre une exécution d'un dispositif de dermabrasion selon l'invention dont l'axe d'oscillation est incliné par rapport au boîtier d'environ 20°, facilitant ainsi certains traitements. A ce fin, la pièce rallongée 32 est coudée et l'élément en U 36 est monté incliné sur le carter 18 au moyen de la vis 44.

Les Figures 5a et 5b montre en coupe deux pièces support-abrasif 32 à double face réversible, comportant d'une part une surface abrasive arquée 30 et, d'autre part, une surface légèrement concave 31a lisse, ou dotée de stries 31b, destinée à un traitement de massage.

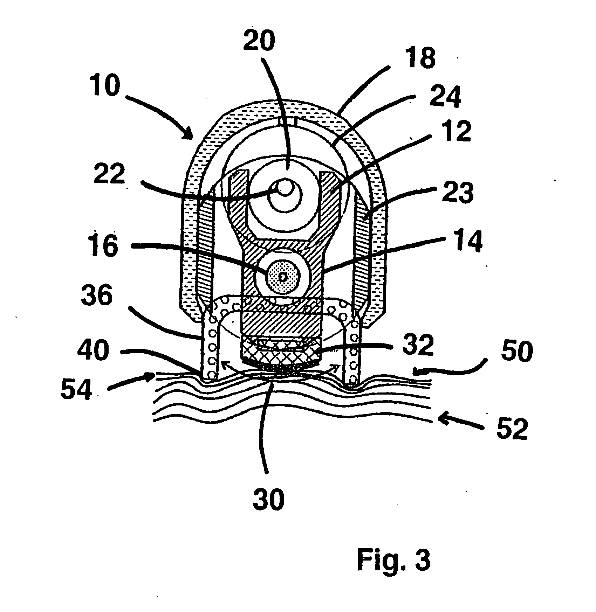

La Figure 6 est un schéma montrant la pièce réversible 32 de la Figure 5b utilisée pour la dermabrasion, dans ce cas avec l'élément en U 36 en place et avec la surface abrasive oscillante 30, entouré de la surface d'appui 40, en contact avec la peau 50, comme dans la Figure 3.

La Figure 7 montre la pièce réversible 32 de la Figure 5b utilisée pour le massage, avec l'élément en U 36 démonté, donc sans surface d'appui 40. Dans ce cas, la pièce 32 est montée à l'envers, c'est-à-dire avec la surface striée de massage 31 en contact avec la peau 50. En outre, dans cette exécution, le moyen d'entraînement du support oscillant 34 (indiqué de manière schématique aux Figures 5a et 5b) est agencé pour animer le support 34 d'un mouvement d'oscillation de va-et-vient perpendiculaire au mouvement d'oscillation, simultanément avec ledit mouvement d'oscillation, comme indiqué par les flèches à la Figure 7. Cette oscillation sera produite par un dispositif connu pour les vibreurs. Ceci permet l'utilisation accessoire du dispositif pour effectuer un massage, notamment après un traitement de dermabrasion.

Le dispositif est de préférence utilisé pour effectuer un traitement de microépidermabrasion après avoir appliqué auparavant à la surface de la peau 50 à traiter une lotion détergente ou autre produit de nettoyage.

La microépidermabrasion au moyen de l'appareil peut être suivi de l'application sur l'épiderme 54 ainsi traité d'un gel ou autre produit traitant, par exemple un gel antirides qu'on fait pénétrer dans le tissu de la peau 50 par l'application d'un flux d'énergie électromagnétique haute fréquence et/ou par l'application de rayonnement électromagnétique laser cohérent, par exemple selon les brevets EP 0573618 et 0773744, ou de la lumière continue dans les couleurs bleu-vert-jaune-rouge de l'arc-en-ciel du spectre visible. Un traitement utilisant une source laser, par exemple selon la demande de brevet WO/9949800, et/ou un LED ou autre source de lumière colorée, est particulièrement commode, notamment un traitement avec l'application successive de deux ou plusieurs sources laser ou lumière à énergies différentes pendant plusieurs minutes. Un tel traitement antirides a donné des résultats spectaculaires après un temps de traitement total de 10 à 20 minutes.

D'autres applications sont le traitement des taches, des vergetures, de l'acné, des cicatrices, d'épilation ou du cuir-chevelu, alors que lorsque la surface d'appui est enlevé d'autres traitements accessoires sont possible par exemple le manucure, le pédicure et le massage.

De manière générale, le traitement de microépidermabrasion améliore de manière significative des traitements ultérieurs. Pour des traitements sur de larges surfaces de la peau, il serait avantageux d'utiliser une surface abrasive disposée sur un cylindre oscillant.

Au lieu d'un moteur rotatif, le moyen d'entraînement pourrait comporter un moteur va-et-vient, comme un vibreur.

## Revendications

1. Dispositif de dermabrasion de la peau par effleurage de la peau avec un abrasif, le dispositif comportant :
- un boîtier maniable (10),
- des moyens d'entraînement en oscillation de l'abrasif, et
- une surface abrasive arquée (30) tenue par un support (34) monté dans ou sur le boîtier (10, 18) pour un mouvement oscillant permettant l'oscillation de la surface abrasive arquée (30),
**caractérisé en ce** :
- la surface abrasive arquée (30) oscille autour de son axe (16), et
- le dispositif comprend une surface d'appui (40) entourant la surface abrasive oscillante (30) sur au moins deux côtés opposés laissant un jeu pour permettre le mouvement oscillant de la surface abrasive (30), le dispositif étant agencé de manière à permettre, par l'application de la surface d'appui (40) contre la peau (50) et autour d'une région de la peau à traiter, l'effleurage de cette région de la peau par la surface abrasive oscillante (30).

2. Dispositif de dermabrasion selon la revendication 1, **caractérisé en ce que** la surface abrasive arquée (30) est au niveau de la surface d'appui (40) ou en retrait par rapport à cette surface.

3. Dispositif de dermabrasion selon la revendication 1 ou 2, **caractérisé en ce que** la surface abrasive (30) est portée sur une pièce (32) en matière rigide ou souple montée amoviblement sur le support oscillant (34).

4. Dispositif de dermabrasion selon la revendication 3, **caractérisé en ce qu'**il comporte plusieurs pièces (32) interchangeables chacune portant une surface abrasive (30) différente et/ou ayant des dimensions différentes.

5. Dispositif de dermabrasion selon la revendication 3 ou 4, **caractérisé en ce qu'**il comporte au moins une pièce amovible (32) à double face montée de manière réversible sur le support oscillant (34).

6. Dispositif de dermabrasion selon la revendication 5, **caractérisé en ce que** la pièce amovible porte une surface abrasive (30) arquée sur une côté et une surface de massage (31a, 31b) sur l'autre côté.

7. Dispositif de dermabrasion selon l'une quelconque des revendication 3 à 6, **caractérisé en ce que** la surface d'appui (40) est constituée par les bords d'un élément en U (36) qui entourent la pièce (32) portant la surface abrasive (30), cette pièce (32) étant amovible par l'extrémité ouverte de l'élément en U (40) de la surface d'appui.

8. Dispositif de dermabrasion selon l'une quelconque des revendications précédentes, **caractérisé en ce que** ladite surface d'appui (40) est constituée par les bords d'un élément (36) monté amoviblement sur le boîtier.

9. Dispositif de dermabrasion selon l'une quelconque des revendications 1 à 7, **caractérisé en ce que** ladite surface d'appui (40) est constituée par les bords du boîtier.

10. Dispositif de dermabrasion selon l'une quelconque des revendications précédentes, **caractérisé en ce que** les moyens d'entraînement (24) permettent la variation de la vitesse d'oscillation de la surface abrasive oscillante (30).

11. Dispositif de dermabrasion selon l'une quelconque des revendications précédentes, **caractérisé en ce que** la surface abrasive oscillante (30) a une vitesse d'oscillation entre 0.5 à 200 oscillations par seconde.

12. Dispositif de dermabrasion selon l'une quelconque des revendications précédentes, **caractérisé en ce que** les moyens d'entraînement comportent un étrier (12) solidaire d'un levier (14) monté pivotant dans le boîtier (10, 18), l'étrier (12) entourant un excentrique (20) entraîné par l'arbre (22) d'un moteur électrique (24), ledit support (32) de la surface abrasive (30) étant monté à l'extrémité du levier (14).

13. Dispositif de dermabrasion selon l'une quelconque des revendications précédentes, **caractérisé en ce que** l'axe d'oscillation de la surface arquée (30) est incliné par rapport à l'axe du boîtier (10).

14. Dispositif de dermabrasion selon l'une quelconque des revendications précédentes, **caractérisé en ce qu'**il comporte un moyen d'entraînement dudit support (34) d'un mouvement d'oscillation de va-et-vient perpendiculaire audit mouvement d'oscillation, simultanément avec ledit mouvement d'oscillation ou au lieu de celui-ci, pour permettre l'utilisation accessoire du dispositif pour effectuer un massage.

15. Dispositif de dermabrasion selon l'une quelconque des revendications 1 à 13, **caractérisé en ce que** le support porte-abrasif (30) est cylindrique et porte au moins une surface abrasive arquée sur sa surface cylindrique.

16. Procédé de traitement cosmétique de la peau par microépidermabrasion, utilisant le dispositif selon l'une quelconque des revendications précédentes.

17. Procédé selon la revendication 16, **caractérisé en ce qu'**on applique auparavant à la surface de la peau à traiter, un produit de nettoyage.

18. Procédé de traitement cosmétique de la peau comportant au préalable une microépidermabrasion selon la revendication 16 ou 17, suivi de l'application sur l'épiderme ainsi traité d'un produit traitant qu'on fait pénétrer dans le tissu de la peau par l'application d'un flux d'énergie électromagnétique haute fréquence et/ou par l'application de rayonnement électromagnétique laser et/ou de la lumière.

19. Procédé selon l'une quelconque des revendications 16 à 18, pour un traitement antirides, des taches, des vergetures, des cicatrices, d'épilation ou du cuir-chevelu.

20. Utilisation du dispositif selon l'une quelconque des revendications 1 à 14 pour la microépidermabrasion de la peau dans un traitement cosmétique.

## Claims

1. Device for skin dermabrasion through gentle contact of the skin with an abrasive, the device comprising :
- a handleable housing (10),
- means for driving the abrasive in oscillation, and
- an arcuate abrasive surface (30) held by a support (34) mounted in or on the housing (10, 18) for an oscillatory motion allowing oscillation of the arcuate abrasive surface (30),
**characterised in that :**
- the arcuate abrasive surface (30) oscillates about its axis (16), and
- the device comprises a support surface (40) surrounding the oscillatory abrasive surface (30) at least on two opposing sides leaving a gap to allow oscillating motion of the abrasive surface (30), the device being arranged in such a way as to allow, by the application of the support surface (40) against the skin (50) and around the region of the skin to be treated, the gentle contact of this region of the skin with the oscillating abrasive surface (30).

2. Dermabrasion device according to claim 1, **characterized in that** the arcuate abrasive surface (30) is at the level of the support surface (40) or inset relative to this surface.

3. Dermabrasion device according to claim 1 or 2, **characterized in that** the abrasive surface (30) is carried on a piece (32) of rigid or flexible material, said piece being removably mounted on the oscillating support (34).

4. Dermabrasion device according to claim 3, **characterized in that** it includes several interchangeable pieces (32) each with a different abrasive surface (30) and/or of a different size.

5. Dermabrasion device according to claim 3 or 4, **characterized in that** it includes at least one removable piece (32) having a double face and mounted in a reversible way on the oscillating support (34).

6. Dermabrasion device according to claim 5, **characterized in that** the removable piece has on one side an arcuate abrasive surface (30) and on the other side a massage surface (31a, 31b).

7. Dermabrasion device according to any of claims 3 to 6, **characterized in that** the support surface (40) is constituted by the edges of a U-shaped element (36) that surround the piece (32) with the abrasive surface (30), this piece (32) being removable through the open end of the U-shaped element (40) of the support surface.

8. Dermabrasion device according to any of the previous claims, **characterized in that** said support surface (40) is constituted by the edges of an element (36) removably-mounted on the housing.

9. Dermabrasion device according to any of claims 1 to 7, **characterized in that** said support surface (40) is constituted by the edges of the housing.

10. Dermabrasion device according to any of the previous claims, **characterized in that** the driving means (24) allow variation of the oscillation speed of the oscillating abrasive surface (30).

11. Dermabrasion device according to any of the previous claims, **characterized in that** the oscillating abrasive surface (30) has an oscillation speed between 0.5 to 200 oscillations per second.

12. Dermabrasion device according to any of the previous claims, **characterized in that** the driving means comprise a stirrup (12) solid with a lever (14) mounted to pivot on the housing (10, 18), the stirrup (12) surrounding a cam (20) driven by the shaft (22) of an electric motor (24), said support (32) of the abrasive surface (30) being mounted at the end of the lever (14).

13. Dermabrasion device according to any of the previous claims, **characterized in that** the oscillation axis of the arcuate surface (30) is inclined to the axis of the housing (10).

14. Dermabrasion device according to any of the previous claims, **characterized in that** it includes means for driving said support (34) with an oscillating motion and to-and-fro motion perpendicular to said oscillating motion, simultaneously with or instead of said oscillating motion, to allow incidental use of the device to carry out a massage.

15. Dermabrasion device according to any of claims 1 to 13, **characterized in that** the support carrying the abrasive (30) is cylindrical and has at least one arcuate abrasive surface on its cylindrical surface.

16. Process for cosmetic skin treatment by microepidermabrasion, using the device according to any of the previous claims.

17. Process according to claim 16, **characterized in that** a cleaning product is applied beforehand to the skin to be treated.

18. Process for cosmetic skin treatment including a preliminary microepidermabrasion according to claim 16 or 17, followed by application on the thus-treated epidermis of a treating product that is made to penetrate the skin tissue by the application of a high-frequency flux of electromagnetic energy and/or by the application of electromagnetic laser radiation and/or by light.

19. Process according to any of claims 16 to 18, for an anti-wrinkle treatment, treatments for blemishes, stretch marks, acne, scars, depilation or for scalp treatment.

20. Use of the device according to any of claims 1 to 14 for skin microepidermabrasion in a cosmetic treatment.

## Patentansprüche

1. Dermabrasionsvorrichtung für die Haut, bei der die Haut mit einem Abrasionsmittel in Kontakt kommt, wobei die Vorrichtung
- ein handliches Gehäuse (10),
- Antriebsmittel für die Oszillation des Abrasionsmittels, und
- eine gewölbte Abrasionsoberfläche (30) aufweist, die für eine oszillierende Bewegung durch einen in oder an dem Gehäuse (10, 18) angebrachten Halter (34) gehalten wird, um die Oszillation der gewölbten Abrasionsoberfläche (30) zu ermöglichen,
**dadurch gekennzeichnet, dass**
- die gewölbte Abrasionsoberfläche (30) um ihre Achse (16) oszilliert und dass
- die Vorrichtung über eine Auflagefläche (40) verfügt, die die oszillierende Abrasionsoberfläche (30) auf wenigstens zwei gegenüberliegenden Seiten umfasst und Spiel für die oszillierende Bewegung der Abrasionsoberfläche (30) lässt, wobei die Vorrichtung dazu eingerichtet ist, durch ein Aufsetzen der Auflagefläche (40) auf die Haut (50) und um einen Bereich der zu behandelnden Haut zu ermöglichen, dass dieser Bereich der Haut mit der oszillierenden Abrasionsoberfläche (30) in Kontakt kommt.

2. Dermabrasionsvorrichtung nach Anspruch 1, **dadurch gekennzeichnet, dass** die gewölbte Abrasionsoberfläche (30) auf einer Ebene mit der Auflagefläche (40) oder im Verhältnis zu dieser Oberfläche zurückversetzt liegt.

3. Dermabrasionsvorrichtung nach Anspruch 1 oder 2, **dadurch gekennzeichnet, dass** die Abrasionsoberfläche (30) auf einem Teil (32) aus einem steifen oder flexiblen Material vorgesehen ist, das entfernbar an dem oszillierenden Halter (34) befestigt ist.

4. Dermabrasionsvorrichtung nach Anspruch 3, **dadurch gekennzeichnet, dass** sie mehrere untereinander austauschbare Teile (32) aufweist, auf denen jeweils eine unterschiedliche und/oder eine unterschiedliche Größe aufweisende Abrasionsoberfläche (30) vorgesehen ist.

5. Dermabrasionsvorrichtung nach Anspruch 3 oder 4, **dadurch gekennzeichnet, dass** sie wenigstens ein entfernbares Teil (32) mit zwei Seiten aufweist, dass lösbar an dem oszillierenden Halter (34) befestigt ist.

6. Dermabrasionsvorrichtung nach Anspruch 5, **dadurch gekennzeichnet, dass** das entfernbare Teil eine gewölbte Abrasionsoberfläche (30) auf der einen Seite und eine Massageoberfläche (31 a, 31 b) auf der anderen Seite aufweist.

7. Dermabrasionsvorrichtung nach einem der Ansprüche 3 bis 6, **dadurch gekennzeichnet, dass** die Auflagefläche (40) durch die Ränder eines U-förmigen Elements (36) gebildet ist, die das Teil (32) umgeben, auf dem die Abrasionsoberfläche (30) vorgesehen ist, wobei dieses Teil (32) durch das offene Ende des U-förmigen Elements (40) der Auflagefläche entfernbar ist.

8. Dermabrasionsvorrichtung nach einem der vorangehenden Ansprüche, **dadurch gekennzeichnet, dass** die Auflagefläche (40) durch die Ränder eines Elements (36) gebildet ist, das entfernbar an dem Gehäuse befestigt ist.

9. Dermabrasionsvorrichtung nach einem der Ansprüche 1 bis 7, **dadurch gekennzeichnet, dass** die Auflagefläche (40) durch die Ränder des Gehäuses gebildet ist.

10. Dermabrasionsvorrichtung nach einem der vorangehenden Ansprüche, **dadurch gekennzeichnet, dass** die Antriebsmittel (24) ein Verändern der Oszillationsgeschwindigkeit der oszillierenden Abrasionsoberfläche (30) ermöglichen.

11. Dermabrasionsvorrichtung nach einem der vorangehenden Ansprüche, **dadurch gekennzeichnet, dass** die oszillierende Abrasionsoberfläche (30) eine Oszillationsgeschwindigkeit zwischen 0,5 und 200 Oszillationen pro Sekunde aufweist.

12. Dermabrasionsvorrichtung nach einem der vorangehenden Ansprüche, **dadurch gekennzeichnet, dass** die Antriebsmittel über einen einstückig mit einem schwenkbar in dem Gehäuse (10, 18) befestigten Hebel (14) ausgebildeten Bügel (12) verfügen, wobei der Bügel (12) einen Exzenter (20) umfasst, der durch die Antriebswelle (22) eines Elektromotors (24) angetrieben wird, und wobei der Halter (32) der Abrasionsoberfläche (30) an dem Ende des Hebels (14) befestigt ist.

13. Dermabrasionsvorrichtung nach einem der vorangehenden Ansprüche, **dadurch gekennzeichnet, dass** die Oszillationsachse der gewölbten Oberfläche (30) in Bezug auf die Achse des Gehäuses (10) geneigt ist.

14. Dermabrasionsvorrichtung nach einem der vorangehenden Ansprüche, **dadurch gekennzeichnet, dass** sie ein Antriebsmittel des Halters (34) für eine pendelnde Oszillationsbewegung senkrecht zu der vorgenannten Oszillationsbewegung aufweist, die gleichzeitig mit der Oszillationsbewegung oder anstatt dieser stattfindet, um eine zusätzliche Verwendung der Vorrichtung zum Durchführen einer Massage zu ermöglichen.

15. Dermabrasionsvorrichtung nach einem der Ansprüche 1 bis 13, **dadurch gekennzeichnet, dass** der das Abrasionsmittel tragende Halter (30) zylindrisch ist und wenigstens eine gewölbte Abrasionsoberfläche auf seiner zylindrischen Oberfläche trägt.

16. Verfahren zur kosmetischen Behandlung der Haut durch Mikroepidermabrasion, bei dem die Vorrichtung nach einem der vorangehenden Ansprüche verwendet wird.

17. Verfahren nach Anspruch 16, **dadurch gekennzeichnet, dass** zuvor auf die zu behandelnde Hautoberfläche ein Reinigungsprodukt aufgetragen wird.

18. Verfahren zur kosmetischen Behandlung der Haut mit einer anfänglichen Mikroepidermabrasion nach Anspruch 16 oder 17 und mit einem anschließendem Auftragen eines Pflegeprodukts auf die so behandelte Epidermis, das durch Anwendung eines hochfrequenten elektromagnetischen Energieflusses und/oder durch Anwendung elektromagnetischer Laserstrahlen und/oder durch Licht in das Gewebe der Haut eingebracht wird.

19. Verfahren nach einem der Ansprüche 16 bis 18 für eine Antifalten-, Flecken-, Schwangerschaftsstreifen-, Narben-, Epilations- oder Kopfhautbehandlung.

20. Verwendung der Vorrichtung nach einem der Ansprüche 1 bis 14 für die Mikroepidermabrasion der Haut bei einer kosmetischen Behandlung.
